# EUROPEAN PATENT SPECIFICATION

(11) **EP 2 813 565 B1**
(45) Date of publication and mention of the grant of the patent: **16.11.2016**
(21) Application number: 13745997.0
(22) Date of filing: 25.01.2013
(51) Int. Cl.: C12M 1/38, C12M 1/12, C12Q 1/68

(54) **ULTRA-HIGH-SPEED NUCLEIC ACID EXTRACTING APPARATUS AND NUCLEIC ACID EXTRACTING METHOD USING SAME**
VORRICHTUNG FÜR ULTRAHOCHGESCHWINDIGKEITSNUCLEINSÄUREEXTRAKTION UND VERFAHREN ZUR NUCLEINSÄUREEXTRAKTION DAMIT
APPAREIL D'EXTRACTION D'ACIDES NUCLÉIQUES À ULTRA-HAUTE VITESSE ET PROCÉDÉ D'EXTRACTION D'ACIDES NUCLÉIQUES L'UTILISANT

(30) Priority: 07.02.2012 KR 20120012222
(43) Date of publication of application: 17.12.2014
(73) Proprietor: Nanobiosys Inc., Seoul 153-712 (KR)
(72) Inventor: KIM, Sung Woo, Seoul 122-904 (KR); KIM, Duck Joong, Anyang-si Gyeonggi-do 431-746 (KR); LEE, Dong Hoon, Anyang-si Gyeonggi-do 430-846 (KR); KIM, Sun Jin, Seoul 120-859 (KR); CHOI, Yong Hea, Seoul 151-883 (KR); RYU, Ho Sun, Seoul 152-896 (KR)
(74) Representative: Gulde & Partner
(86) International application number: PCT/KR2013/000609
(87) International publication number: WO 2013/118990

(56) References cited:
- WO-A1-2010/009415
- WO-A1-2011/066588
- WO-A2-2008/089493
- WO-A2-2010/147654
- JP-A- 2009 125 033
- JP-A- 2012 018 039
- KR-A- 20090 124 311
- KR-A- 20110 035 476
- KR-B1- 100 441 893
- KR-B1- 101 082 348
- US-A1- 2008 257 071
- US-A1- 2010 216 244

## Description

### TECHNICAL FIELD

The present invention relates to a device for extracting a nucleic acid from a biological specimen, such as sputum, blood, cells, urine, saliva, tissues, etc.

### BACKGROUND ART

For diagnosis, treatment, or prevention of diseases at the genetic level, techniques for extracting nucleic acids from a biological specimen such as cells, bacterium, or viruses have recently been in wide use in association with the nucleic acid amplification techniques. The techniques for extracting nucleic acids from a biological specimen are also on demand in various fields of applications, such as customized drug development, forensic, detection of endocrine disruptors, and so forth, in addition to the diagnosis, treatment, or prevention of diseases.

An example of the conventional nucleic acid extraction techniques is a method of solubilizing a specimen including cells with SDS or proteinase K, modifying and removing proteins with phenol and then purifying a nucleic acid. However, the phenol-based extraction method not only requires a number of steps to take a lot of time but also has the efficiency of nucleic acid extraction greatly influenced by the worker's experience and skills, adversely bring about a great deterioration of the reliability. For solving this problem, there has recently been used a kit using silica or glass fiber that specifically combines with a nucleic acid. The silica or glass fiber has a low combining ratio with proteins or cell metabolites, so it is possible to acquire a nucleic acid at a relatively high concentration. This method is advantageously more convenient in comparison to the phenol-based method. But the method uses a chaotropic reagent or ethanol that strongly inhibits the enzyme reaction such as polymerization chain reaction (PCR) or the like and thus requires a complete removal of the substances, that is, the chaotropic reagent or ethanol, so it is too onerous to work and takes too much time. Recently, International Laid-Open Patent No. 00/21973 discloses a method of directly purifying a nucleic acid with a filter, which method involves passing a specimen through a filter to have cells adsorbed by the filter, dissolving the adsorbed cells, filtering the cells, and then washing and eluting the adsorbed nucleic acid. It is, however, necessary to select the filter depending on the type of the cells in order to adsorb the cells with the filter and then elute the nucleic acid. Further, the devices used in this method are too large and complicated for the worker to use with ease.

### DISCLOSURE OF INVENTION

For solving the problems with the conventional nucleic acid extraction techniques, the present invention is to provide a nucleic acid extraction device that is capable of realizing automation, ultra-miniaturization and super-high speed unlike the existing nucleic acid extraction device and method, having no limitation to the biological specimens, such as sputum, blood, cells, urine, saliva, tissues, etc., minimizing the used amount of the sample solution, and also maintaining and/or improving the nucleic acid extraction efficiency with reliability.

In accordance with one embodiment of the present invention, there is provided a device for extracting a nucleic acid from a biological specimen that includes: a substrate; a chip support being arranged on the top surface of the substrate and including a chip receiving portion having the shape of a plate with a horizontal surface in close contact with a bottom surface of a microfluidic chip for nucleic acid extraction; and a heater being arranged on the horizontal surface of the chip receiving portion and made to apply heat to a part of the whole bottom surface of the microfluidic chip for nucleic acid extraction installed in the chip receiving portion.

In one embodiment of the present invention, the heater may be a contact plate type heating block.

Further, the device may further include a chip cover being arranged on the top of the chip support and having a horizontal surface in close contact with the top surface of the microfluidic chip for nucleic acid extraction to be installed in the chip receiving portion.

Further, the device may further include a fluid control module made to control the flow of a reactant solution received in the microfluidic chip for nucleic acid extraction installed in the chip receiving portion.

Further, the device may further include a heat control module connected to the heater to control the heat capacity provided for a reactant solution received in the microfluidic chip for nucleic acid extraction installed in the chip receiving portion.

Further, the device may further include a microfluidic chip for nucleic acid extraction having the shape of a plate with a top horizontal surface and a bottom horizontal surface, where the microfluidic chip for nucleic acid extraction includes: an inlet portion; a heating portion being arranged in a first channel region connected to the inlet portion and made to transfer heat obtained from an exterior to a biological specimen introduced through the inlet portion; a first filter being arranged in a second channel region connected to the heating portion and allowing a substance having an equivalent size of the nucleic acid to pass through; a nucleic acid separating portion being arranged in a third channel region connected to the first filter and having a nucleic acid binding substance capable of specifically binding with the nucleic acid; a second filter being arranged in a fourth channel region connected to the nucleic acid separating portion and allowing a substance having an equivalent size of the nucleic acid to pass through; and an outlet portion connected to the second filter.

Further, a channel including the first to fourth channel regions of the microfluidic chip for nucleic acid extraction may be made to allow a fluid to pass through and have a width and a depth in the range of 0.001 to 10 mm.

Further, the first and second filters of the microfluidic chip for nucleic acid extraction may have a pore with a diameter in the range of 0.1 to 0.4 µm and a thickness in the range of 0.01 to 10 mm.

Further, the first and second filters of the microfluidic chip for nucleic acid extraction may have a pore having a diameter of 0.2 µm and a thickness of 0.01 to 0.5 mm.

Further, the nucleic acid separating portion of the microfluidic chip for nucleic acid extraction may have a bead with a nucleic acid binding functional group on the surface thereof as a nucleic acid binding substance.

Further, the bead having a nucleic acid binding functional group of the microfluidic chip for nucleic acid extraction may have a diameter in the range of 0.001 to 20 mm.

Further, the nucleic acid separating portion of the microfluidic chip for nucleic acid extraction may include the bead having a nucleic acid binding functional group in an amount of 1 µg to 200 mg.

Further, the microfluidic chip for nucleic acid extraction may be made of a plastic material.

Further, the microfluidic chip for nucleic acid extraction may include a first plate; a second plate being arranged on the first plate and having a channel including the first to fourth channel regions; and a third plate being arranged on the second plate and having the input portion and the output portion.

Further, the first and third plates of the microfluidic chip for nucleic acid extraction may include a material selected from the group consisting of polydimethylsiloxane (PDMS), cycloolefin copolymer (COC), polymethylmethacrylate (PMMA), polycarbonate (PC), polypropylene carbonate (PPC), polyether sulfone (PES), polyethylene terephthalate (PET), and a mixture thereof. The second plate of the microfluidic chip for nucleic acid extraction may include a thermoplastic resin or thermosetting resin material selected from the group consisting of polymethylmethacrylate (PMMA), polycarbonate (PC), cycloolefin copolymer (COC), polyamide (PA), polyethylene (PE), polypropylene (PP), polyphenylene ether (PPE), polystyrene (PS), polyoxymethylene (POM), polyetheretherketone (PEEK), polytetrafluoroethylene (PTFE), polyvinylchloride (PVC), polyvinylidene fluoride (PVDF), polybutyleneterephthalate (PBT), fluorinated ethylenepropylene (FEP), perfluoroalkoxyalkane (PFA), and a mixture thereof.

Further, the inlet portion of the third plate of the microfluidic chip for nucleic acid extraction may have a diameter in the range of 0.1 to 5.0 mm. The outlet portion of the microfluidic chip for nucleic acid extraction may have a diameter of 0.1 to 5.0 mm. The first and third plates of the microfluidic chip for nucleic acid extraction may have a thickness in the range of 0.01 to 20 mm. The second plate of the microfluidic chip for nucleic acid extraction may have a thickness in the range of 30 µm to 10 mm.

### EFFECTS OF THE INVENTION

According to the nucleic acid extraction device of the present invention, it is possible to realize automation, ultra-miniaturization and super-high speed in the nucleic acid extraction, have no limitation to the biological specimens, such as sputum, blood, cells, urine, saliva, tissues, etc., minimize the used amount of the sample solution, and also maintain and/or improve the nucleic acid extraction efficiency with reliability.

### BRIEF DESCRIPTION OF DRAWINGS

FIG. 1 is an illustration of a nucleic acid extraction device according one embodiment of the present invention.
FIG. 2 is an illustration of the nucleic acid extraction device according to one embodiment of the present invention with a chip cover.
FIG. 3 is an illustration of the nucleic acid extraction device according to one embodiment of the present invention with a fluid control module.
FIG. 4 is an illustration of the nucleic acid extraction device according to one embodiment of the present invention with a heat control module.
FIG. 5 is an illustration of a microfluidic chip for nucleic acid extraction according to one embodiment of the present invention.
FIG. 6 is a cross-sectional plane view of the microfluidic chip for nucleic acid extraction according to one embodiment of the present invention.
FIG. 7 is an illustration that the microfluidic chip for nucleic acid extraction of FIG. 5 is installed in the nucleic acid extraction device according to one embodiment of the present invention.

### BEST MODES FOR CARRYING OUT THE INVENTION

Hereinafter, one embodiment of the present invention will be described with reference to the accompanying drawings. The description is given for the better understanding of the embodiments of the present invention and not intended to limit the scope of the present invention.

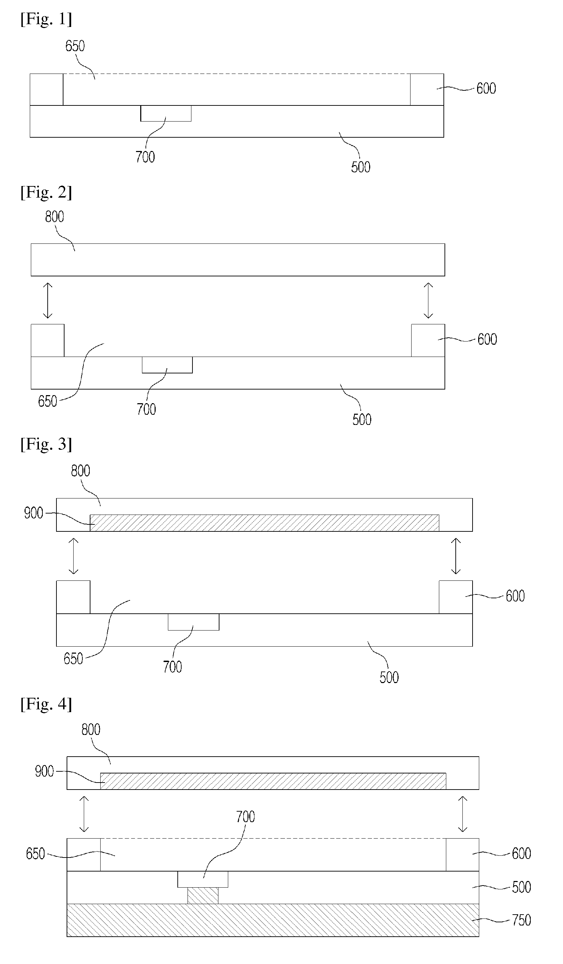

FIG. 1 is an illustration of a nucleic acid extraction device according one embodiment of the present invention.

According to FIG. 1, the nucleic acid extraction device according to one embodiment of the present invention for extracting a nucleic acid from a biological specimen includes: a substrate 500; a chip support 600 being arranged on the top surface of the substrate 500 and including a chip receiving portion 650 having the shape of a plate with a horizontal surface in close contact with a bottom surface of a microfluidic chip for nucleic acid extraction (not shown); and a heater 700 being arranged on the horizontal surface of the chip receiving portion 650 and made to apply heat to a part of the whole bottom surface of the microfluidic chip for nucleic acid extraction installed in the chip receiving portion 650.

The nucleic acid extraction device means a device made to perform all the steps for nucleic acid extraction and, of course, may further include different modules required to extract a nucleic acid.

The substrate 500 may be made of any material that has no change in the physical and/or chemical properties by heating with the heater 700 after-mentioned that is arranged in the substrate 500 or by maintaining the temperature and does not allow deformation on the portions other than the heater in the substrate 500 by thermal conduction. The substrate 500 may be made of, if not limited to, plastic, glass, silicon, or the like and appear transparent or semitransparent. The substrate 500 may be made to have different shapes and preferably made in the form of a plate having a horizontal surface as shown in FIG. 1.

The chip support 600 is a part on which the microfluidic chip for nucleic acid extraction according to the present invention mentioned afterward in detail is mounted and fixed. Thus, the chip support 600 may be arranged on the top surface of the substrate 500 and, of course, made of a material capable of mounting and fixing the microfluidic chip for nucleic acid extraction (not shown) in a stable way. Further, the chip support 600 has the plate-shaped chip receiving portion 650 with a horizontal surface in close contact with the bottom surface of the microfluidic chip for nucleic acid extraction (not shown). As the chip receiving portion 650 has the shape of a plate, it gets in close contact with the bottom surface of the microfluidic chip for nucleic acid extraction. This increases the contact area of the heat transferred from the heater 700 mentioned afterward in detail and raises the heat transfer rate to enable a stable fluid control of the reactant solution received in the microfluidic chip for nucleic acid extraction, so the nucleic acid extraction reaction can be achieved even with a small amount of the reactant solution.

The heater 700 is a module for supplying heat for a part of the microfluidic chip for nucleic acid extraction (not shown) when the microfluidic chip for nucleic acid is mounted on the chip support 600. The heater 700 may be differently realized but it is preferably a contact plate type heating block in order to increase the heat transfer rate. In one embodiment of the present invention, the heater 700 may be arranged on the horizontal surface of the chip receiving portion 600 and made to apply heat to a part of the whole bottom surface of the microfluidic chip for nucleic acid extraction installed in the chip receiving portion 600.

FIG. 2 is an illustration of the nucleic acid extraction device according to one embodiment of the present invention with a chip cover.

According to FIG. 2, the nucleic acid extraction device according to one embodiment of the present invention may further include a chip cover 800 being arranged on the top of the chip support 600 and having a horizontal surface in close contact with the top surface of the microfluidic chip for nucleic acid extraction (not shown) to be installed in the chip receiving portion 650. The chip cover 800 supports to stably mount the microfluidic chip for nucleic acid extraction and prevents emission of heat from the microfluidic chip for nucleic acid extraction, so that the nucleic acid extraction reaction can take place rapidly in the microfluidic chip for nucleic acid extraction. The chip cover 800 may be made in different shapes for achieving the above-described object and is preferably made in the form of a plate.

FIG. 3 is an illustration of the nucleic acid extraction device according to one embodiment of the present invention with a fluid control module.

According to FIG. 3, the nucleic acid extraction device according to one embodiment of the present invention may further include a fluid control module 900 made to control the flow of a reactant solution received in the microfluidic chip for nucleic acid extraction installed in the chip receiving portion 650. The fluid control module 900 is connected to the inlet portion and/or the outlet portion of the microfluidic chip for nucleic acid extraction installed on the chip support 600, so it can be made to introduce a solution for nucleic acid extraction into the microfluidic chip for nucleic acid extraction and/or exhaust the solution from the inside of the microfluidic chip for nucleic acid extraction and/or to control the movement of the reagent solution inside the microfluidic chip for nucleic acid extraction. The fluid control module 900 may include various components. For example, the fluid control module 900 may further include a micro-channel as a fluid passage, a pneumatic pump for providing a driving force for fluid flow, a valve for controlling the fluid flow, or a storage chamber containing different solutions required to extract a nucleic acid, such as a nucleic acid binding buffer, an elution buffer, silica gel, distilled water (DW), etc.

FIG. 4 is an illustration of the nucleic acid extraction device according to one embodiment of the present invention with a heat control module.

Referring to FIG. 4, the nucleic acid extraction device according to one embodiment of the present invention may further include a heat control module 750 connected to the heater 700 to control the heat capacity provided for a reactant solution received in the microfluidic chip for nucleic acid extraction installed in the chip receiving portion 650. By controlling the power supply for the heater 700, the heat control module 750 can control the heating or cooling of the heater 700 so that the nucleic acid extraction reaction can occur according to the temperature and the sequential order as preset in the microfluidic chip for nucleic acid extraction. On the other hand, if not shown in FIG. 4, the nucleic acid extraction device according to one embodiment of the present invention may further include an electronic control module (not shown) to automatically control the heater 700, the cover 800, the fluid control module 900, and the heat control module 750. The electronic control module can control the individual modules precisely to precisely extract a predetermined amount of the nucleic acid in the microfluidic chip for nucleic acid extraction according to a saved program. The saved program includes, for example, a program related to a series of steps concerning the nucleic acid extraction.

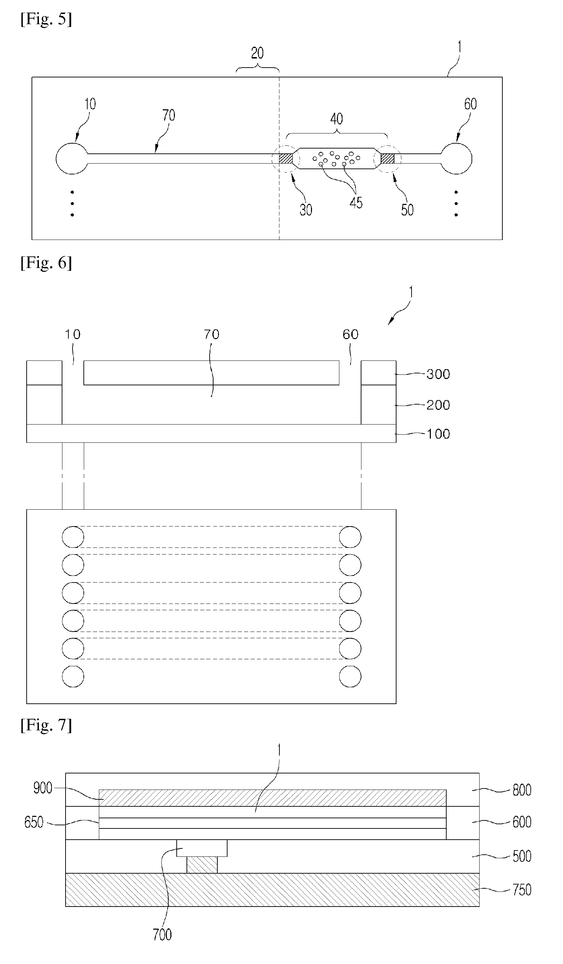

FIG. 5 is an illustration of the microfluidic chip for nucleic acid extraction according to one embodiment of the present invention.

Referring to FIG. 5, the microfluidic chip 1 for nucleic acid extraction according to one embodiment of the present invention, which is to extract a nucleic acid from a biological specimen, includes: an inlet portion 10; a heating portion 20 being arranged in a first channel region connected to the inlet portion 10 and made to transfer the heat obtained from an exterior to the biological specimen introduced through the inlet portion 10; a first filter 30 being arranged in a second channel region connected to the heating portion 20 and allowing a substance having an equivalent size of the nucleic acid to pass through; a nucleic acid separating portion 40 being arranged in a third channel region connected to the first filter 30 and having a nucleic acid binding substance 45 capable of specifically binding with the nucleic acid; a second filter 50 being arranged in a fourth channel region connected to the nucleic acid separating portion 40 and allowing a substance having an equivalent size of the nucleic acid to pass through; and an outlet portion 60 connected to the second filter 50.

The microfluidic chip for nucleic acid extraction as used herein refers to a microchip in which the components for nucleic acid extraction, that is, the inlet portion, the outlet portion, the channel connecting the inlet portion with the outlet portion, the first filter, the second filter, and so forth are realized in the size on a millimeter (mm) or micrometer (µm) scale.

The biological specimen as used herein is a biological substance including a nucleic acid such as DNA or RNA. For example, the biological specimen may be a liquid specimen containing, if not limited to, animal cells, plant cells, pathogenic bacteria, fungi, bacteria, viruses, etc.

The inlet portion 10 is a portion through which the biological specimen or the solution for nucleic acid extraction is introduced into the microfluidic chip. The outlet portion 60 is a portion through which the nucleic acid acquired from the biological specimen, the solution for nucleic acid extraction, or other wastes are exhausted from the microfluidic chip. In this case, the inlet portion 10 and the outlet portion 60 may play a role as a inlet or an outlet, respectively. The solution for nucleic acid extraction includes any solution required in extracting a nucleic acid. Examples of the solution for nucleic acid extraction may include distilled water, a nucleic acid binding buffer, an elution buffer, and so forth. On the other hand, the inlet portion 10 and the outlet portion 60 are connected with each other via a channel 70 so that a fluid can move between them. The components after-mentioned, such as the heating portion 20, the first filter 30, the nucleic acid separating portion 40, and the second filter 50, are arranged in the channel 70 so that they can be driven to perform their respective functions. The channel 70 may be made in different sizes, but the width and the depth of the channel 70 are preferably in the range of 0.001 to 10 mm. The first, second, third, and fourth channel regions after-mentioned are given merely in terms of the sequential arrangement from the inlet portion 10 to the outlet portion 60 and not limited to specific locations in the channel 70.

The heating portion 20 is a portion in which the heat obtained from the exterior is applied on the solution (including the biological specimen) introduced through the inlet portion 10. The heating portion 20 is arranged in the first channel region connected to the inlet portion 10. For example, when a specimen including cells, bacteria or viruses is introduced through the inlet portion 10, the cells, bacteria or viruses upon arriving at the heating portion 20 are instantly heated to about 80 to 100 °C and their outer cell membranes are destroyed to release the contents of the disrupted cells. The heating portion 20 is supplied with the heat by means of the contact from the heater 70 of the nucleic acid extraction device according to one embodiment of the present invention.

The first filter 30 is a structure having pores of a predetermined size and used to separate the substances according to their size and identify the substances that pass through the pores in the direction of the fluid from the substances that cannot pass through the pores. In one embodiment of the present invention, the first filter 30 is arranged in the second channel region connected to the heating portion 20 and designed to allow the substances having an equivalent size of the nucleic acid to pass through. Out of the dissolution product prepared by the heating in the heating portion 20, the first filter 30 collects the substances larger than the nucleic acid in the heating portion 20 and allows the nucleic acid and the substances having the size of the nucleic acid to pass through and move to the nucleic acid separating portion 40 after-mentioned. The first filter 30 may be made in different sizes, but preferably with pores having a diameter in the range of 0.1 to 0.4 µm and a thickness in the range of 0.01 to 0.5 mm.

The nucleic acid separating portion 40 is to selectively separate the nucleic acid from the substances having an equivalent size of the nucleic acid. Referring to FIG. 5, the nucleic acid separating portion 40 is a space between the first filter 30 and the second filter 50 after-mentioned and has the nucleic acid binding substance 45 capable of specifically binding with the nucleic acid. The nucleic acid binding substance 45 is a bead with a nucleic acid binding functional group and may be, for example, a silica bead or a biotin or streptavidin bead. The bead having a nucleic acid binding functional group may be made in different sizes, but preferably with a diameter in the range of 0.001 to 20 mm. In addition, the nucleic acid separating portion 40 may contain the bead having a nucleic acid binding functional group in different amounts. But, the content of the bead having a nucleic acid binding functional group in the nucleic acid separating portion 40 is preferably in the range of 1 µg to 200 mg. After the nucleic acid is specifically bound to the nucleic acid binding substance 45, the inside of the nucleic acid separating portion 40 is washed to eliminate foreign substances, leaving a complex of the target nucleic acid and the nucleic acid binding substance 45 only in the nucleic acid separating portion 40. Subsequently, an elution buffer is applied to the nucleic acid separating portion 40 to separate the target nucleic acid from the complex.

Like the first filter 30 aforementioned, the second filter is a structure having pores of a predetermined size and used to separate the substances according to their size and identify the substances that pass through the pores in the direction of the fluid from the substances that cannot pass through the pores. In one embodiment of the present invention, the second filter 50 is arranged in the fourth channel region connected to the nucleic acid separating portion 40 and designed to allow the substances having an equivalent size of the nucleic acid to pass through. The second filter 50 collects the substances larger than the nucleic acid in the nucleic acid separating portion 40 and allows the nucleic acid separated from the nucleic acid binding substance 45 to pass through and move to the outlet portion 60. The second filter 50 may be made in different sizes, but preferably with pores having a diameter in the range of 0.1 to 0.4 µm and a thickness in the range of 0.01 to 0.5 mm. More preferably, the second filter 50 has pores having a diameter of 0.2 µm and a thickness of 0.3 mm.

FIG. 6 is a cross-sectional plane view of the microfluidic chip for nucleic acid extraction according to one embodiment of the present invention.

According to FIG. 6, the microfluidic chip 1 for nucleic acid extraction according to one embodiment of the present invention includes: a first plate 100; a second plate 200 being arranged on the first plate 100 and having a channel 70 including the first to fourth channel regions; and a third plate 300 being arranged on the second plate 200 and having the input portion 10 and the output portion 60. The microfluidic chip 1 for nucleic acid extraction may be formed of different materials but is preferably made of a plastic material. When the microfluidic chip 1 for nucleic acid extraction is made of a plastic material, it is possible to increase the heat transfer efficiency merely by adjusting the thickness of the plastic and to greatly reduce the production cost with the simple production process. On the other hand, the first and third plates 100 and 300 may include a material selected from the group consisting of polydimethylsiloxane (PDMS), cycloolefin copolymer (COC), polymethylmethacrylate (PMMA), polycarbonate (PC), polypropylene carbonate (PPC), polyether sulfone (PES), polyethylene terephthalate (PET), and a mixture of these. The second plate 200 may include a thermoplastic resin or thermosetting resin material selected from the group consisting of polymethylmethacrylate (PMMA), polycarbonate (PC), cycloolefin copolymer (COC), polyamide (PA), polyethylene (PE), polypropylene (PP), polyphenylene ether (PPE), polystyrene (PS), polyoxymethylene (POM), polyetheretherketone (PEEK), polytetrafluoroethylene (PTFE), polyvinylchloride (PVC), polyvinylidene fluoride (PVDF), polybutyleneterephthalate (PBT), fluorinated ethylenepropylene (FEP), perfluoroalkoxyalkane (PFA), and a mixture of these. Further, the inlet portion of the third plate has a diameter in the range of 0.1 to 5.0 mm. The outlet portion of the third plate has a diameter of 0.1 to 5.0 mm. The first and third plates have a thickness in the range of 0.01 to 20 mm and the second plate has a thickness in the range of 30 µm to 10 mm. In addition, under necessity, the microfluidic chip for nucleic acid extraction according to one embodiment of the present invention may include at least two inlet portions, at least two outlet portions, and channels connecting them together, in which case it is possible to extract the nucleic acid from at least two biological specimens on a single chip, thereby achieving extraction of the nucleic acid in a rapid and efficient way.

FIG. 7 is an illustration that the microfluidic chip for nucleic acid extraction of FIG. 5 is installed in the nucleic acid extraction device according to one embodiment of the present invention.

According to FIG. 7, the nucleic acid extraction reaction can be carried out while the plate-shaped microfluidic chip 1 for nucleic acid extraction is in close contact with the chip receiving portion 650 of the nucleic acid extraction device according to one embodiment of the present invention. More specifically, the method for extracting a nucleic acid from a biological specimen using the nucleic acid extraction device according to one embodiment of the present invention includes: a step (microfluidic chip feeding step) of feeding the microfluidic chip 1 for nucleic acid extraction according to one embodiment of the present invention; a step (biological specimen introducing step) of introducing a biological specimen, such as sputum, blood, cells, urine, saliva, tissues, etc., through the inlet portion of the microfluidic chip 1; a step (biological specimen lysis step) of transferring the introduced biological specimen to the heating portion of the microfluidic chip 1 and then applying heat to the heating portion of the microfluidic chip 1 through the heater 700 to dissolve the biological specimen; a step (filtration step using the first filter) of transferring the substance obtained from the lysis step to the first filter of the microfluidic chip 1, passing the substance through the first filter and then eliminating the substances that cannot pass through the first filter; a step (nucleic acid separation step) of transferring the substances passing through the first filter to the nucleic acid separating portion of the microfluidic chip 1, binding the nucleic acid among the substances passing through the first filter with the nucleic acid binding substance and then eliminating the substances not binding with the nucleic acid binding substance; a step (filtration step using the second filter) of isolating the nucleic acid from the nucleic acid binding substance, transferring the isolated nucleic acid to the second filter and then passing the nucleic acid through the second filter; and a step (nucleic acid extraction step) of transferring the substances passing through the second filter and then extracting the nucleic acid through the outlet portion. Therefore, the use of the nucleic acid extraction device according to the present invention can realize automation, ultra-miniaturization and super-high speed in the nucleic acid extraction reaction, have no limitation to the biological specimens, such as sputum, blood, cells, urine, saliva, tissues, etc., minimize the used amount of the sample solution, and maintain and/or improve the nucleic acid extraction efficiency with reliability.

### Experimental Example: Yield of nucleic acid extraction and required time

Firstly, a general nucleic acid extraction device using a tube and a nucleic acid extraction device using a microfluidic chip for nucleic acid extraction according to one embodiment of the present invention are independently used to extract DNAs from a cell of the tubercular bacillus strain, and the yield of the DNAs and the required time are determined.

The general nucleic acid extraction step is as follows. The cell of the tubercular bacillus strain is prepared and mixed with 6% NaOH and 4% NaLC at a mixing ratio of 1:1:1 to prepare a sample solution. The sample solution is subjected to a centrifugal separation and removed of the supernatant (20 min, 4,300 rpm, 4°C). 1 ml of distilled water (DW) is added to the sample solution and, after vortexing, the sample solution is transferred to another tube. The sample solution is subjected to a second centrifugal separation and then removed of the supernatant (3 min, 12,000 rpm, room temp.). 1 ml of distilled water (DW) is added to the sample solution and, after vortexing, 500 µl of distilled water (DW) is added to the sample solution to obtain a DNA (using a QIAamp DNA Kit commercially available). As a result, the yield of the final product is about 100 µl and the required time to obtain the final DNA product is at least about one hour.

Subsequently, the microfluidic chip 1 for nucleic acid extraction and the nucleic acid extraction device according to one embodiment of the present invention are used to extract the nucleic acid from the same cell of the tubercular bacillus strain. The specific procedures are as follows. The cell of the tubercular bacillus strain is prepared and mixed with 6% NaOH and 4% NaLC at a mixing ratio of 1:1:1 to prepare a sample solution. Then, a syringe is used to introduce the sample solution into the inlet portion of the microfluidic chip for nucleic acid extraction (25x72x2 mm³, silica bead (OPS Diagnostics, LLC), filter (Whatman)) of FIG. 1 (about one minute required). Into the inlet portion of the microfluidic chip according to one embodiment of the present invention are introduced silica gel and 300 µl of a 1X DNA binding buffer. And, the heating portion of the microfluidic chip according to one embodiment of the present invention is rapidly heated up to 95 °C (about one minute and thirty seconds required). Through the inlet portion of the microfluidic chip according to one embodiment of the present invention, the wastes out of the sample solution are eliminated and 100 µl of an elution buffer is introduced (about 30 seconds required). Then, the final product is obtained through the outlet portion of the microfluidic chip according to one embodiment of the present invention. As a result, the yield of the final DNA product is about 100 µl and the required time to obtain the final DNA product is about 7 minutes. This shows that the use of the automatic nucleic acid extraction device rather than the manual nucleic acid extraction device in the experiment can shorten the required time to about 5 minutes or less.

As a result of the experiment, compared to the conventional nucleic acid extraction method, the microfluidic chip and the nucleic acid extraction device according to one embodiment of the present invention can be used to greatly reduce the required time for the nucleic acid extraction while maintaining the yield of the nucleic acid extraction.

## Claims

1. A device for extracting a nucleic acid from a biological specimen, comprising:
a substrate (500);
a chip support (600) being arranged on the top surface of the substrate (500) and including a chip receiving portion (650) having the shape of a plate with a horizontal surface in close contact with a bottom surface of a microfluidic chip for nucleic acid extraction;
a heater (700) in form of a contact plate type heating block, the heater (700) being arranged on the horizontal surface of the chip receiving portion (650) and made to apply heat to a part of the whole bottom surface of the microfluidic chip (1) for nucleic acid extraction installed in the chip receiving portion (650); anda microfluidic chip (1) for nucleic acid extraction having the shape of a plate with a top horizontal surface and a bottom horizontal surface, wherein the microfluidic chip (1) comprises:
an inlet portion (10) to receive a biological specimen;
an outlet portion (60) to retrieve extracted nucleic acid;
a channel (70) connecting the inlet portion (10) and the outlet portion (60);
a first filter (30) and a second filter (50), wherein the first filter (30) and the second filter (50) are arranged within the channel (70) and wherein the first filter (30) and the second filter (50) are configured to allow a substance having an equivalent size of a nucleic acid to pass through;
a nucleic acid separating portion (40), wherein said nucleic acid separating portion (40) is located between the first filter (30) and the second filter (50), and wherein said nucleic acid separating portion (40) comprises a nucleic acid binding substance (45) capable of specifically binding nucleic acid; and
a heating portion (20), wherein the heating portion (20) is arranged within the channel (70) and is situated between the inlet portion (10) and the first filter (30), wherein the heating portion (20) is configured to transfer heat obtained from the heater (700) to a biological specimen introduced through the inlet portion (10).

2. The device according to claim 1, further comprising:
a chip cover (800) being arranged on the top of the chip support (600) and having a horizontal surface in close contact with the top surface of the microfluidic chip (1) for nucleic acid extraction to be installed in the chip receiving portion (650).

3. The device according to claim 1, further comprising:
a fluid control module (900) made to control the flow of a reactant solution received in the microfluidic chip (1) for nucleic acid extraction installed in the chip receiving portion.

4. The device according to claim 1, further comprising:
a heat control module (750) connected to the heater (700) to control the heat capacity provided for a reactant solution received in the microfluidic chip (1)for nucleic acid extraction installed in the chip receiving portion.

5. The device according to claim 1, wherein the channel (70) of the microfluidic chip (1) is made to allow a fluid to pass through and have a width and a depth in the range of 0.001 to 10 mm.

6. The device according to claim 1, wherein the first filter (30) and the second filter (50) of the microfluidic chip (1) for nucleic acid extraction have a pore with a diameter in the range of 0.1 to 0.4 µm and a thickness in the range of 0.01 to 0.5 mm.

7. The device according to claim 6, wherein the first filter (30) and the second filter(50) of the microfluidic chip (1) for nucleic acid extraction have a pore having a diameter of 0.2 µm and a thickness of 0.01 to 0.5 mm.

8. The device according to claim 1, wherein the nucleic acid separating portion (40) of the microfluidic chip (1) for nucleic acid extraction has a bead with a nucleic acid binding functional group on the surface thereof as a nucleic acid binding substance (45).

9. The device according to claim 8, wherein the bead having a nucleic acid binding functional group of the microfluidic chip (1) for nucleic acid extraction has a diameter in the range of 0.001 to 20 mm.

10. The device according to claim 8, wherein the nucleic acid separating portion (40) of the microfluidic chip (1) for nucleic acid extraction comprises the bead having a nucleic acid binding functional group in an amount of 1 µg to 200 mg.

11. The device according to claim 1, wherein the microfluidic chip (1) for nucleic acid extraction comprises a first plate (100); a second plate (200) being arranged on the first plate (100) and having the channel (70) ; and a third plate (300) being arranged on the second plate (200) and having the inlet portion (10) and the outlet portion (60).

12. The device according to claim 11, wherein the inlet portion (10) of the third plate (300) of the microfluidic chip (1) for nucleic acid extraction has a diameter in the range of 0.1 to 5.0 mm, wherein the outlet portion (60) of the microfluidic chip (1) for nucleic acid extraction having has a diameter of 0.1 to 5.0 mm, wherein the first plate (100) and the third plate (300) of the microfluidic chip (1) for nucleic acid extractionhave a thickness in the range of 0.01 to 20 mm, wherein the second plate (200) of the microfluidic chip (1) for nucleic acid extraction has a thickness in the range of 30 µm to 10 mm.

## Patentansprüche

1. Vorrichtung zur Extraktion einer Nucleinsäure aus einer biologischen Probe, umfassend:
ein Substrat (500);
einen Chipträger (600), der auf der Oberseite des Substrates (500) angeordnet ist und einen Chipaufnahmeteil (650) umfasst, der die Form einer Platte mit einer waagerechten Fläche in engem Kontakt mit einer Unterseite eines mikrofluidischen Chips zur Nucleinsäureextraktion aufweist;
eine Heizvorrichtung (700) in Form eines Kontaktplatten-Heizblocks, wobei die Heizvorrichtung (700) auf der waagerechten Fläche des Chipaufnahmeteils (650) angeordnet ist und so ausgelegt ist, dass sie einem Teil der gesamten Unterseite des in den Chipaufnahmeteil (650) eingesetzten mikrofluidischen Chips (1) zur Nucleinsäureextraktion Wärme zuführt; und einen mikrofluidischen Chip (1) zur Nucleinsäureextraktion, der die Form einer Platte mit einer waagerechten Oberseite und einer waagerechten Unterseite aufweist, wobei der mikrofluidische Chip (1) umfasst:
einen Einlassteil (10) zum Aufnehmen einer biologischen Probe;
einen Auslassteil (60) zum Entnehmen extrahierter Nucleinsäure;
einen Kanal (70), der den Einlassteil (10) und den Auslassteil (60) verbindet;
einen ersten Filter (30) und einen zweiten Filter (50), wobei der erste Filter (30) und der zweite Filter (50) in dem Kanal (70) angeordnet sind und wobei der erste Filter (30) und der zweite Filter (50) so ausgelegt sind, dass sie eine Substanz, der eine einer Nucleinsäure entsprechende Größe aufweist, passieren lassen;
einen Nucleinsäureabtrennungsteil (40), wobei sich der Nucleinsäureabtrennungsteil (40) zwischen dem ersten Filter (30) und dem zweiten Filter (50) befindet, und wobei der Nucleinsäureabtrennungsteil (40) eine nucleinsäurebindende Substanz (45) umfasst, die Nucleinsäure spezifisch binden kann; und
einen Heizteil (20), wobei der Heizteil (20) in dem Kanal (70) angeordnet ist und sich zwischen dem Einlassteil (10) und dem ersten Filter (30) befindet, wobei der Heizteil (20) so ausgelegt ist, dass er aus der Heizvorrichtung (700) erhaltene Wärme auf eine durch den Einlassteil (10) hindurch eingebrachte biologische Probe überträgt.

2. Vorrichtung nach Anspruch 1, weiterhin umfassend:
eine Chipabdeckung (800), die oben auf dem Chipträger (600) angeordnet ist und
eine waagerechte Fläche in engem Kontakt mit der Oberseite des in den Chipaufnahmeteil (650) einzusetzenden mikrofluidischen Chips (1) zur Nucleinsäureextraktion aufweist.

3. Vorrichtung nach Anspruch 1, weiterhin umfassend:
ein Flüssigkeitsregelungsmodul (900), das so ausgelegt ist, dass es den Strom einer Reaktantenlösung regelt, die in dem in den Chipaufnahmeteil eingesetzten mikrofluidischen Chip (1) zur Nucleinsäureextraktion aufgenommen wird.

4. Vorrichtung nach Anspruch 1, weiterhin umfassend:
ein mit der Heizvorrichtung (700) verbundenes Wärmeregelungsmodul (750) zum Regeln der Wärmekapazität, die für eine Reaktantenlösung bereitgestellt wird, die in dem in den Chipaufnahmeteil eingesetzten mikrofluidischen Chip (1) zur Nucleinsäureextraktion aufgenommen wird.

5. Vorrichtung nach Anspruch 1, wobei der Kanal (70) des mikrofluidischen Chips (1) so ausgelegt ist, dass er eine Flüssigkeit passieren lässt und eine Breite und eine Tiefe im Bereich von 0,001 bis 10 mm aufweist.

6. Vorrichtung nach Anspruch 1, wobei der erste Filter (30) und der zweite Filter (50) des mikrofluidischen Chips (1) zur Nucleinsäureextraktion eine Pore mit einem Durchmesser im Bereich von 0,1 bis 0,4 µm und eine Dicke im Bereich von 0,01 bis 0,5 mm aufweisen.

7. Vorrichtung nach Anspruch 6, wobei der erste Filter (30) und der zweite Filter (50) des mikrofluidischen Chips (1) zur Nucleinsäureextraktion eine Pore mit einem Durchmesser von 0,2 µm und eine Dicke von 0,01 bis 0,5 mm aufweisen.

8. Vorrichtung nach Anspruch 1, wobei der Nucleinsäureabtrennungsteil (40) des mikrofluidischen Chips (1) zur Nucleinsäureextraktion ein Kügelchen mit einer nucleinsäurebindenden funktionellen Gruppe auf dessen Oberfläche als nucleinsäurebindende Substanz (45) aufweist.

9. Vorrichtung nach Anspruch 8, wobei das Kügelchen mit einer nucleinsäurebindenden funktionellen Gruppe des mikrofluidischen Chips (1) zur Nucleinsäureextraktion einen Durchmesser im Bereich von 0,001 bis 20 mm aufweist.

10. Vorrichtung nach Anspruch 8, wobei der Nucleinsäureabtrennungsteil (40) des mikrofluidischen Chips (1) zur Nucleinsäureextraktion das Kügelchen mit einer nucleinsäurebindenden funktionellen Gruppe in einer Menge von 1 µg bis 200 mg umfasst.

11. Vorrichtung nach Anspruch 1, wobei der mikrofluidische Chip (1) zur Nucleinsäureextraktion umfasst: eine erste Platte (100); eine zweite Platte (200), die auf der ersten Platte (100) angeordnet ist und den Kanal (70) aufweist; und eine dritte Platte (300), die auf der zweiten Platte (200) angeordnet ist und den Einlassteil (10) und den Auslassteil (60) aufweist.

12. Vorrichtung nach Anspruch 11, wobei der Einlassteil (10) der dritten Platte (300) des mikrofluidischen Chips (1) zur Nucleinsäureextraktion einen Durchmesser im Bereich von 0,1 bis 5,0 mm aufweist, wobei der Auslassteil (60) des mikrofluidischen Chips (1) zur Nucleinsäureextraktion einen Durchmesser von 0,1 bis 5,0 mm aufweist, wobei die erste Platte (100) und die dritte Platte (300) des mikrofluidischen Chips (1) zur Nucleinsäureextraktion eine Dicke im Bereich von 0,01 bis 20 mm aufweisen, wobei die zweite Platte (200) des mikrofluidischen Chips (1) zur Nucleinsäureextraktion eine Dicke im Bereich von 30 µm bis 10 mm aufweist.

## Revendications

1. Dispositif pour extraire un acide nucléique d'un spécimen biologique, comprenant:
un substrat (500) ;
un support de puce (600) étant disposé sur la surface supérieure du substrat (500) et
comprenant une portion accueillant la puce (650) ayant la forme d'une plaque avec une surface horizontale en contact étroit avec une surface inférieure d'une puce microfluidique pour l'extraction d'acide nucléique;
un élément chauffant (700) sous la forme d'un bloc chauffant de type plaque de contact, l'élément chauffant (700) étant disposé sur la surface horizontale de la portion accueillant la puce (650) et fait pour appliquer de la chaleur à une partie de la surface inférieure totale de la puce microfluidique (1) pour l'extraction d'acide nucléique installée dans la portion accueillant la puce (650) ; et une puce microfluidique (1) pour l'extraction d'acide nucléique ayant la forme d'une plaque avec une surface horizontale supérieure et une surface horizontale inférieure, la puce microfluidique (1) comprenant :
une portion d'entrée (10) pour recevoir un spécimen biologique;
une portion de sortie (60) pour recueillir l'acide nucléique extrait;
un canal (70) reliant la portion d'entrée (10) et la portion de sortie (60);
un premier filtre (30) et un deuxième filtre (50), le premier filtre (30) et le deuxième filtre (50) étant disposés dans le canal (70), et le premier filtre (30) et le deuxième filtre (50) étant configurés pour permettre à une substance ayant une taille équivalente à celle d'un acide nucléique de passer ;
une portion de séparation d'acide nucléique (40), ladite portion de séparation d'acide nucléique (40) étant située entre le premier filtre (30) et le deuxième filtre (50), et
ladite portion de séparation d'acide nucléique (40) comprenant une substance liante d'acide nucléique (45) capable de lier spécifiquement l'acide nucléique; et
une portion de chauffage (20), la portion de chauffage (20) étant disposée dans le canal (70) et étant située entre la portion d'entrée (10) et le premier filtre (30), la portion de chauffage (20) étant configurée pour transmettre la chaleur obtenue par l'élément chauffant (700) à un spécimen biologique introduit par la portion d'entrée (10).

2. Dispositif selon la revendication 1, comprenant en outre:
une protection de puce (800) étant disposée sur le dessus du support de puce (600) et ayant une surface horizontale en contact étroit avec la surface supérieure de la puce microfluidique (1) pour l'extraction d'acide nucléique devant être installée dans la portion accueillant la puce (650).

3. Dispositif selon la revendication 1, comprenant en outre:
un module de contrôle de fluide (900) fait pour contrôler l'écoulement d'une solution réactive accueillie dans la puce microfluidique (1) pour l'extraction d'acide nucléique installée dans la portion accueillant la puce.

4. Dispositif selon la revendication 1, comprenant en outre:
un module de contrôle de chaleur (750) relié à l'élément chauffant (700) pour contrôler la capacité thermique fournie par la solution réactive accueillie dans la puce microfluidique (1) pour l'extraction d'acide nucléique installée dans la portion accueillant la puce.

5. Dispositif selon la revendication 1, le canal (70) de la puce microfluidique (1) étant fait pour permettre à un fluide de passer et ayant une largeur et une profondeur dans la plage de 0,001 à 10 mm.

6. Dispositif selon la revendication 1, le premier filtre (30) et le deuxième filtre (50) de la puce microfluidique (1) pour l'extraction d'acide nucléique ayant un pore avec un diamètre dans la plage de 0,1 à 0,4 µm et une épaisseur dans la plage de 0,01 à 0,5 mm.

7. Dispositif selon la revendication 6, le premier filtre (30) et le deuxième filtre (50) de la puce microfluidique (1) pour l'extraction d'acide nucléique ayant un pore avec un diamètre de 0,2 µm et une épaisseur de 0,01 à 0,5 mm.

8. Dispositif selon la revendication 1, la portion de séparation d'acide nucléique (40) de la puce microfluidique (1) pour l'extraction d'acide nucléique ayant une perle avec un groupe fonctionnel liant d'acide nucléique sur la surface de celle-ci en tant que substance liante d'acide nucléique (45).

9. Dispositif selon la revendication 8, la perle ayant un groupe fonctionnel liant d'acide nucléique de la puce microfluidique (1) pour l'extraction d'acide nucléique ayant un diamètre dans la plage de 0,001 à 20 mm.

10. Dispositif selon la revendication 8, la portion de séparation d'acide nucléique (40) de la puce microfluidique (1) pour l'extraction d'acide nucléique comprenant la perle ayant un groupe fonctionnel liant d'acide nucléique dans une quantité de 1 µg à 200 mg.

11. Dispositif selon la revendication 1, la puce microfluidique (1) pour l'extraction d'acide nucléique comprenant une première plaque (100) ; une deuxième plaque (200) étant arrangée sur la première plaque (100) et ayant le canal (70) ; et une troisième plaque (300) étant arrangée sur la deuxième plaque (200) et ayant la portion d'entrée (10) et la portion de sortie (60).

12. Dispositif selon la revendication 11, la portion d'entrée (10) de la troisième plaque (300) de la puce microfluidique (1) pour l'extraction d'acide nucléique ayant un diamètre dans la plage de 0,1 à 5,0 mm, la portion de sortie (60) de la puce microfluidique (1) pour l'extraction d'acide nucléique ayant un diamètre de 0,1 à 5,0 mm, la première plaque (100) et la troisième plaque (300) de la puce microfluidique (1) pour l'extraction d'acide nucléique ayant une épaisseur dans la plage de 0,01 à 20 mm, la deuxième plaque (200) de la puce microfluidique (1) pour l'extraction d'acide nucléique ayant une épaisseur dans la plage de 30 µm à 10 mm.
